# EUROPÄISCHE PATENTSCHRIFT

(11) **EP 2 280 691 B1**
(45) Veröffentlichungstag und Bekanntmachung des Hinweises auf die Patenterteilung: **03.08.2022**
(21) Anmeldenummer: 09757163.2
(22) Anmeldetag: 13.05.2009
(51) Int. Cl.: A61K 47/24, C30B 7/00, C30B 7/08, C30B 29/58, C07F 9/10, A61K 9/107, A61P 17/00, A61P 43/00

(54) **kristallines DOPC**
crystalline DOPC
DOPC cristallin

(30) Priorität: 06.06.2008 EP 08010331
(43) Veröffentlichungstag der Anmeldung: 09.02.2011
(73) Patentinhaber: Merck Patent GmbH, 64293 Darmstadt (DE)
(72) Erfinder: PLATSCHER, Michael, CH-8252 Schlatt (CH); HEDINGER, Alfred, CH-8240 Thayngen (CH)
(86) Internationale Anmeldenummer: PCT/EP2009/003398
(87) Internationale Veröffentlichungsnummer: WO 2009/146779

(56) Entgegenhaltungen:
- US-A1- 2006 067 998
- HISHIDA ET AL: "Stacking structures of dry phospholipid films on a solid substrate" COLLOIDS AND SURFACES. A, PHYSICACHEMICAL AND ENGINEERING ASPECTS, ELSEVIER, AMSTERDAM, NL, Bd. 284-285, 15. August 2006 (2006-08-15), Seiten 444-447, XP005496833 ISSN: 0927-7757
- Bjoern A. Bergenstaahl ET AL: "Phase diagrams of dioleoylphosphatidylcholine with formamide, methylformamide and dimethylformamide", JOURNAL OF PHYSICAL CHEMISTRY, vol. 91, no. 23, 1 November 1987 (1987-11-01), pages 5944-5948, XP055408019, US ISSN: 0022-3654, DOI: 10.1021/j100307a026

## Beschreibung

Die vorliegende Erfindung betrifft kristallines DOPC, Verfahren zu deren Herstellung sowie deren Verwendung zur Herstellung von pharmazeutischen Zusammensetzungen.

Mit DOPC sind oben und im Folgenden das natürlich vorkommende 1,2-Dioleoyl-sn-glycero-3-phosphocholine, auch als 1,2-Dioleoyl-sn-glycero-3-phosphatidylcholine, 1,2-Dioleoyl-sn-glycero-3-phosphorylcholine, (R)-2,3-Bis(oleoyloxy)propyl-2-(trimethylammonio)ethyl phosphate, 1,2-Dioleoyl-L-α-lecithin oder, (R)-DOPC bezeichnet dessen natürliches nicht vorkommendes Enantiomer 2,3-Dioleoyl-sn-glycerol-1-phosphocholine, racemisches (R,S)-DOPC und andere Mischungen der oben genannten Enantiomere gemeint. 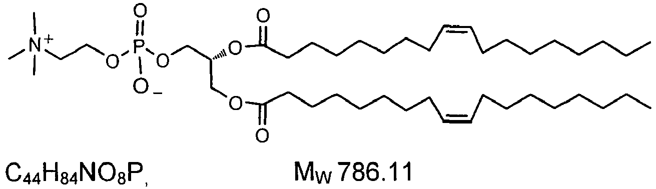 CAS Nummern: 4235-95-4 (R-Form) und 84366-67-6 (S-Form) Liposomen sind künstliche mehrschichtige Vesikel (kugelig in sich geschlossene Membranen) aus ambiphilen Substanzen, meist natürlichen Lipiden, in die sowohl hydrophile Substanzen in den wässrigen Innenraum verkapselt, als auch lipophile Substanzen in den Innenbereich der Lipidmembran inkorporiert werden können.

Sie werden vor allem in der Kosmetik und in der Medizin, speziell in der Dermatologie eingesetzt. Dabei werden vor allem Vitamine, Coenzyme, Haut- und Sonnenschutzmittel eingebettet. Liposomen werden in der Regel topisch angewendet.

In der pharmazeutischen Technologie gewinnen Liposomen aber zunehmend weitere Bedeutung, da durch die parenterale Applikation von Liposomen eine spezifischere Organverteilung erzielt werden kann, als wenn die Wirkstoffe in frei gelöster Form angewendet werden.

So ist aus US 2006/0067998 A1ein Methode bekannt, worin zur Behandlung oder Vorbeugung von Krebserkrankungen einer colloidal verkapselten Formulierung verabreicht wird, welche Kurkumin oder ein Kurkuminderivat als aktive Substanz enthält. Geeignete Formulierungen enthalten sowohl Lipid- basierte colloidiale Systeme als auch polymere colloidale Systeme, wie beispielsweise Liposomen, Nanopartikel, Mikropartikel oder aus Blockpolymeren gebildete Copolymer-Micellen.

Wenn man DNA-, RNA- oder Proteine einschliesst werden Lipoplexe erhalten.

Bei Nanopartikeln (Nanoparts) handelt es sich um Partikel etwa gleicher Größenordnung wie Liposomen, die aber in ihrem Inneren keine Wasserphase, sondern eine Ölphase oder einen festen Kern besitzen. Sie eignen sich insbesondere für die Verkapselung von lipophilen Substanzen.

Mikroemulsionen sind kolloiddisperse, einphasige Systeme aus wässrigen, lipidartigen und tensidartigen Komponenten. Sie weisen eine Teilchengrösse von 1-500 nm auf und verhalten sich ähnlich wie Flüssigkeiten.

Gerade im Zusammenhang mit den normalerweise schlecht löslichen peptidartigen Wirkstoffen, Nukleotiden, Vakzinen und anderen Biopharmazeutika hat der lösungsvermittelnde Effekt bei den oben beschriebenen Anwendungen eine sehr grosse Bedeutung.

Ausserdem lässt sich auf diese Weise der Abbau der Wirkstoffe im Körper bremsen und ein Sustained-Release-Effekt erzielen.

(R)-DOPC gehört zu der Klasse der natürlich vorkommenden, zwitterionischen Phosphocholine. Liposomen aus zwitterionischen Lipiden weisen, allein oder kombiniert mit anderen Phosphocholinen oder anderen ungeladenen, lipidartigen Verbindungen, eine neutrale Oberfläche auf.

Besonders wichtig ist jedoch die Fähigkeit von DOPC basierten Liposomen und Lipoplexen, in Zellen eindringen zu können und somit die in sie eingeschlossenen Wirkstoffe in das Zellinnere zu transportieren (Transfektion). Solche Liposomen enthalten oft geladene Lipide, insbesondere kationische Lipide. Es sind aber auch Anwendungen publiziert, bei denen die Lipoplexe ausschliesslich aus DOPC bestehen, etwa durch Sood et al. in the Journal of the National Cancer Institute (2008),100, 359 - 372.

All diese Eigenschaften machen DOPC auch sehr interessant für die Krebstherapie. Es bietet sich durch diese Eigenschaften die Möglichkeit, in DOPC-Liposomen eingeschlossene RNA, DNA oder herkömmliche Cytostatika zu applizieren.

Medizinische, insbesondere parenterale Anwendungen stellen höchste Anforderungen an die Qualität und Reinheit der verwendeten Wirk- und Hilfsstoffe. So gibt es von behördlicher Seite sehr strenge Vorschriften bezüglich der Herstellung, der Reproduzierbarkeit der Herstellung sowie des Nebenproduktprofils dieser Verbindungen. Bei parenteral angewendeten Substanzen kommt noch hinzu, dass mikrobiologische Verunreinigungen durch pathogene Keime und Endotoxine strikt zu vermeiden und zu kontrollieren sind.

DOPC ist bei Raumtemperatur instabil und daher an sich schwierig in einer akzeptablen Reinheit herzustellen, so dass es sich für die Verwendung zur Herstellung einer Arzneimittelformulierung eignet.

Wie alle Lipide, die Ölsäurereste tragen, wie beispielsweise die natürlichen Phospholipide POPC und DOPE, ist DOPC sehr oxidationsempfindlich. Die Oxidationsprodukte ungesättigter Fettsäurederivate weisen jedoch in der Regel eine hohe Toxizität auf.

Hier sind geeignete Herstell- und Reinigungsmethoden gefordert. DOPC liegt beispielsweise als Lyophilisat oder wachsartiger Feststoff vor und ist deshalb technisch nur sehr schwer in hinreichender Qualität zu erhalten.

Die üblichen Methoden zur Überwindung der Instabilität, wie z. B. die Zugabe von Oxidationsschutzmitteln in Form von Tocopherol oder reduziertem L-Glutathion, schränken die generelle Anwendbarkeit von DOPC stark ein, weil Interaktionen mit den später einzubettenden Wirkstoffen nicht auszuschliessen sind. Ein vollständiger Sauerstoffausschluss während der Herstellung, Lagerung und Verwendung ist kaum zu verwirklichen, bzw. nur mit sehr grossem Aufwand zu ermöglichen.

So wird für lyophilisiertes DOPC in der Regel vom Hersteller eine Lagerung unter Schutzgas bei -20°C empfohlen und dabei nur eine Haltbarkeit von ca. 12 Monaten garantiert. Für wachsartiges DOPC wird zwar eine längere Haltbarkeit garantiert, jedoch ist ein Oxidationsschutz zugesetzt, und die Lagerung muss ebenfalls bei -20°C erfolgen.

Sowohl lyophilisiertes als auch wachsartiges DOPC weisen sehr hohe amorphe Anteile auf.

Dieses amorphe DOPC ist neben seiner Oxidatidationsempfindlichkeit auch noch extrem hygroskopisch und zerläuft bei normaler Luftfeuchtigkeit innerhalb kürzester Zeit zu einem schmierigen Film. Ausserdem ist wachsartiges DOPC nur schwer zu zerkleinern und wie auch lyophilisiertes DOPC nur schwer abzuwiegen. Dies erschwert die Handhabbarkeit dieser Verbindung enorm.

Aus der Literatur sind lediglich verschiedene Synthesenwege für die Herstellung von amorphem DOPC bekannt:
Ichihara et al., Chemistry and Physics of Lipids (2005), 137 (1-2), 94-99, schildern die Synthese von DOPC aus SN-Glycero-3-phosphocholine (GPC).

Roodsari et al., Journal of Organic Chemistry (1999), 64(21), 7727-7737, beschreiben die Totalsynthese von DOPC ausgehend von Tritylglycerin.

Viele andere Publikationen zur Synthese und Verwendung von DOPC sind erschienen, jedoch keine beschreibt kristallines Material.

Lewis et al., Biochemistry (1988), 27(3), 880-7, sowie Biochemistry (1989), 28(2), 541-8, berichten über lyophilisiertes DOPC.

Baer und Kindler sprechen in Biochemistry (1962), 1(3), 518-21, von wachsartigem DOPC. In US2006067998 ist die Herstellung von kristallinen DOPC gezeigt.

Lekim, Biedermann und Ghyczy, DE 2647395, beschreiben generisch Aufreinigung von GPC-Estern durch Kristallisation, die explizite Kristallisation von DOPC wird jedoch nicht beschrieben.

Racemisches DOPC kann aus racemischen Edukten analog der für die Enantiomeren beschriebenen Verfahren erhalten werden.

Auch ist in keiner der vielen Publikationen ein Schmelzpunkt für DOPC genannt.

Aufgabe der vorliegenden Erfindung ist es daher, DOPC in hoher Reinheit, möglichst in kristalliner Form zur Verfügung zu stellen. Eine weitere Aufgabe der vorliegenden Erfindung ist die Bereitstellung dieser Verbindung mit hoher Lagerstabilität und Handhabbarkeit, so dass sie zur Herstellung pharmazeutischer Formulierungen einsetzbar ist. Weiterhin besteht ein starkes Bedürfnis nach einem reproduzierbaren, in technischem Maßstab durchführbaren Prozess zur Herstellung von stabilen Formen von DOPC.

Durch Versuche wurde nun überraschend gefunden, dass sich sowohl racemisches, als auch enantiomerenreines, kristallines DOPC mit einer hohen chemischen Reinheit und einer ausgezeichneten Stabilität in einfacher Weise erhalten lassen. Die so erhaltenen kristallinen Produkte sind bei Raumtemperatur unter Schutzgas praktisch unbeschränkt stabil. Auch fällt kristallines DOPC als gut handhabbares Schüttgut mit verbesserter Hygroskopizität an.

Sie sind daher als Bestandteil oder als Ausgangsmaterial zur Herstellung von Arzneimittelformen geeignet.

Gegenstand der vorliegenden Erfindung sind demnach stabile Kristallmodifikationen von DOPC-Enantiomeren, sowie Mischungen der Enantiomeren mit derselben Kristallform.

Die stabilen Kristallmodifikationen können in kristalliner und teilkristalliner Form vorliegen. Sie weisen eine bisher nie erreichte Reinheit von > 98% zusammen mit einer bisher nie erreichten Stabilität von > 99% in Bezug auf den Ausgangswert nach 12 Monaten Lagerung unter Luftausschluss bei 40°C und 75% relativer Luftfeuchtigkeit und nach 18 Monaten bei 25°C und 60% relativer Luftfeuchtigkeit auf (ohne Zusatz von Oxidationsschutz, siehe dazu Tabelle 1). Die DOPC Kristallmodifikationen haben einen Gehalt von weniger als 1 Äquivalent Wasser oder Kristalllösungsmittel auf 1 Äquivalent DOPC.

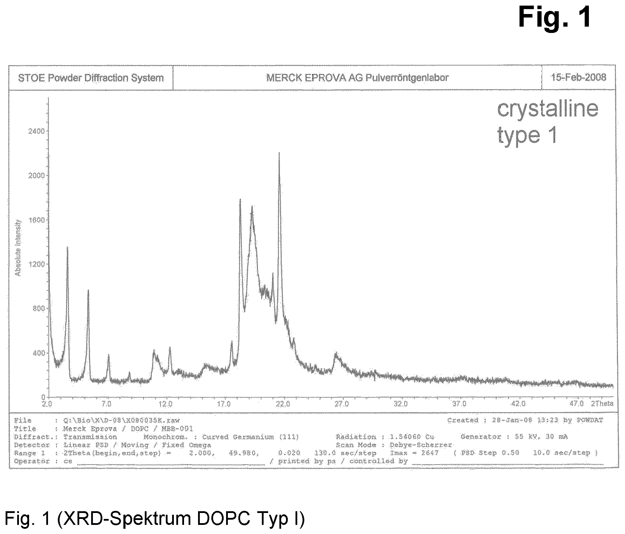

Enantiomerenreines DOPC liegt beispielsweise in der Kristallmodifikation vom Typ I vor und zeigen bei Pulverröntgendiffraktions-Messungen mittelscharfe Banden (siehe dazu Abb. 1 und Tabelle 2). Ausgewählte 2-Theta-Werte für die verschiedenen Kristallmodifikationen liegen bei 3.6, 5.3, 18.3, 19.3, und 21.7 (Typ I), gemessen mit Cu-Kα-Strahlung. Geringe Abweichungen einzelner Banden von diesen Werten können auftreten, wenn unterschiedliche Geräte oder Aufnahmemethoden wie Reflektion oder Transmission, beziehungsweise Kapillare oder Fenster, gewählt werden, oder wenn unterschiedliche Aufnahmebedingungen bezüglich Luftfeuchtigkeit oder Temperatur herrschen.

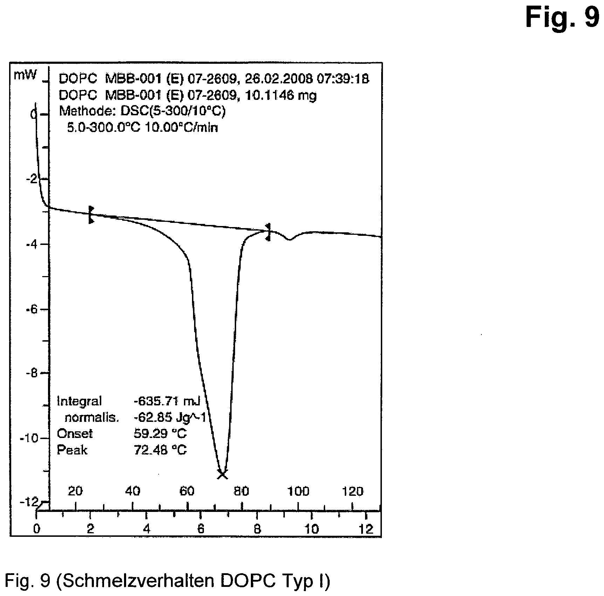

Die Kristallmodifikationen weisen teilweise einen sehr hohen kristallinen Anteil auf, der sich in einer Schmelzenthalpie grösser als 48 J/g ausdrückt. Der Schmelzpunkt der Kristallmodifikationen liegt in der Regel über 60°C (Abb. 9).

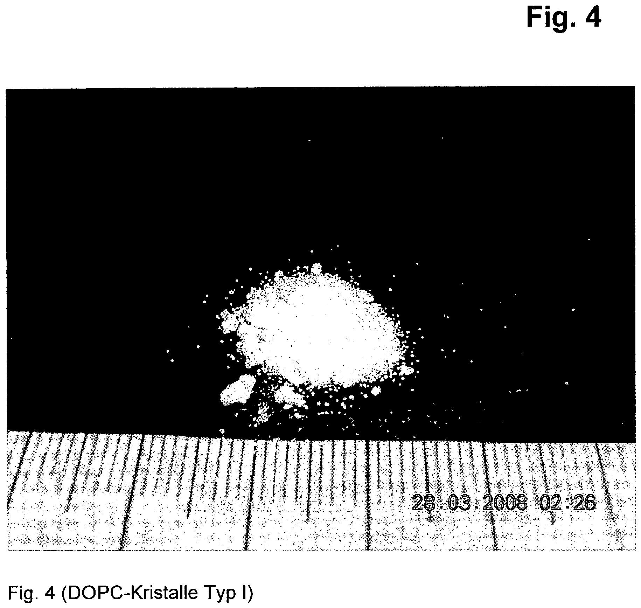

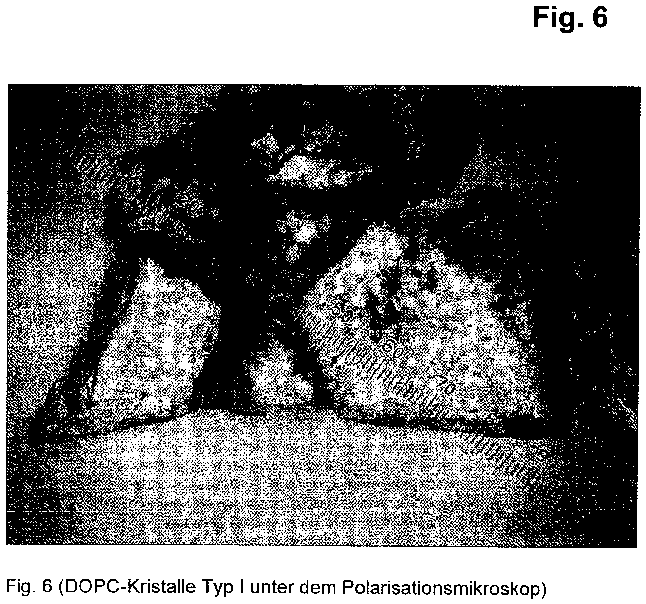

Ausserdem fallen die erfindungsgemässen Kristallmodifikationen als gut handhabbares Schüttgut an (Abb. 4). Der kristalline Charakter und wird unter dem Polarisationsmikroskop deutlich (Abb. 6).

Auch Mischungen der kristallinen DOPC-Enantiomeren, wie etwa kristallines Racemat, können die gleichen XRD-Spektren aufweisen.

Ein weiterer Gegenstand der Erfindung ist ein Verfahren zur Herstellung von DOPC Kristallmodifikationen, welches dadurch gekennzeichnet ist, dass man DOPC aus einem aprotischen Medium auskristallisiert. Als aprotisches Medium können zu diesem Zweck aprotische Lösungsmittel oder deren Mischungen verwendet werden. Das aprotische Medium kann auch in geringem Umfang protische Lösungsmittel, wie z. B. Wasser, enthalten. In Ausnahmefällen können unter geeigneten Bedingungen auch 25 Gew.-% protische Lösungsmittel enthalten sein. Die Kristallisation von DOPC kann hierbei ohne vorgängige Aufreinigung direkt aus der Reaktionslösung erfolgen. Ebenso kann kristallines DOPC durch Umkristallisation von amorphem, teilkristallinem oder kristallinem Material gewonnen werden

Als aprotische Lösungsmittel eignen sich vor allem
- Ether, wie z. B.: Tetrahydrofuran, Methyltetrahydrofuran und Dioxan,
- Ester, wie z. B.: Ethylformiat, Methylacetat, Ethylacetat, Propylacetat, Isopropylacetat, Butylacetat, Isobutylacetat, Dimethylcarbonat, Diethylcarbonat und 1,3- Dioxolidin-2-on,
- Ketone,: wie z. B. Aceton, 2-Butanon, Methylisobutylketon, Methylisopropylketon und
- Nitrile, wie z. B.: Acetonitril.

Protische Lösungsmittelzusätze bestehen typischerweise aus
- Alkoholen wie z. B.: Methanol, Ethanol, n-Propanol, iso-Propanol, n-Butanol, iso-Butanol, 2-Butanol, tert-Butanol, 3-Methyl-1-butanol und Ethylenglycol, Methoxy-ethanol, Ethoxyethanol,

Wasser
oder Gemischen daraus.

Die Kristallisation der DOPC Modifikationen wird in der Regel gezielt durch langsames Abkühlen der hergestellten Lösung auf Temperaturen unter 30°C erreicht. Die Bildung der Kristalle erfolgt entweder spontan oder durch Animpfen mit der entsprechenden DOPC Kristallmodifikation.

Die verschiedenen DOPC Kristallmodifikationen lassen sich ineinander umwandeln. Die Umwandlungen können durch Temperaturbehandlungen der isolierten Kristallmodifikationen bei erhöhter Temperatur oder durch länger andauerndes Rühren ihrer Suspensionen unter Kristallisationsbedingungen erreicht werden.

Durch den Einsatz von amorphem oder teilkristallinem DOPC als Ausgangsmaterial für die Umkristallisierung erhält man durch das beschriebene Verfahren im Wesentlichen kristallines DOPC von bisher nie erreichter Reinheit zusammen mit einer bisher nie erreichten Stabilität.

Die Erfindung betrifft auch die Verwendung von kristallinem DOPC zur Herstellung von Arzneimittelformulierungen, da kristallines DOPC unter den gegebenen Bedingungen in fester Form eine ausgezeichnet Stabilität besitzt und eine zeitlich praktisch unbeschränkt eine gleichbleibende und sehr gute Qualität aufweist.

Ein weiterer Gegenstand der Erfindung sind folglich auch die aus der Verwendung der beanspruchten DOPC Formen resultierenden pharmazeutischen Zusammensetzungen. Diese können beispielsweise als Liposomen, Lipoplexe, Mikroemulsionen und Nanopartikel vorliegen und beispielsweise einen Wirkstoff aus der Gruppe der Peptide, Nukleotide, Vakzine oder Zytostatika beinhalten.

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann. Die vorliegende Beschreibung ermöglicht es folglich dem Fachmann, die Erfindung umfassend anzuwenden und auszuführen.

Zum besseren Verständnis und zur Verdeutlichung der Erfindung werden im folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Die in den Beispielen und der Beschreibung sowie in den Ansprüchen gegebenen Temperaturen gelten immer in °C. Wo nicht anders bezeichnet sind Gehaltsangaben als Gew.-% aufgeführt.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten %-Angaben als Gew.-%, mit Ausnahme von Verhältnissen, die in Volumenangaben wiedergegeben sind.

### Beispiele zur Illustrierung der Erfindung

### Beispiel 1

### Kristallisation von (R)-DOPC

200 g amorphes (R)-DOPC werden bei 25°C in 1700 ml Acetonitril gelöst. Die Lösung wird mit 0.1°C/Min auf -10°C abgekühlt. Bei 10°C tritt Kristallisation ein. Nach abgeschlossener Kristallisation wird das Produkt durch Filtration isoliert und im Vakuum getrocknet. Die Ausbeute an kristallinem (R)-DOPC beträgt 180 g (90%).

In gleicher Weise lassen sich die Kristallmodifikationen (R,S)- und (S)-DOPC erhalten.

### Beispiel 1a

### Umkristallisation von DOPC aus Ethylacetat

20.0 g DOPC werden bei 35°C in 100 ml Ethylacetat gelöst. Die Lösung wird rasch auf 20°C und danach mit einer Rampe von 0.01°C/min auf - 10°C abgekühlt, wobei Kristallisation eintritt. Die Kristallisat wird abfiltriert und im Vakuum bei Raumtemperatur getrocknet. Die Ausbeute beträgt 19.6 g kristallines DOPC (98.1% d. Th.). Das Produkt zeigt bei einer Heizrate von 5°C/min einen Schmelzpunkt von 71°C und eine Schmelzenthalpie von 49.4 J/g

### Beispiel 2

### Stabilitäten

Zur Stabilitätsbestimmung des kristallinen DOPC werden die Substanzen zusammen mit Vergleichsmustern bei 25°C und 60% relativer Feuchte sowie bei 40°C und 75% relativer Feuchte unter Luftausschluss gelagert. In periodischen Abständen wird der verbleibende Gehalt an DOPC gemessen und im Vergleich zum Ausgangswert angegeben.

Reinheit und Gehalt an DOPC werden mit HPLC bestimmt.

Für Kristallmodifikation Typ I wurden folgende Werte gefunden:

**Tabelle 1:**

| Belastungszeit in Monaten | **Stabilität von (R)-DOPC kristallin Typ** I | | | |
|---|---|---|---|---|
| | 25°C / 60% relative Feuchte | | 40°C / 75% relative Feuchte | |
| | Gewichts% | Flächen% | Gewichts% | Flächen% |
| 0 | 100.0 | 100.0 | 100.0 | 100.0 |
| 3 | 100.9 | 100.0 | 100.0 | 99.9 |
| 6 | 99.3 | 100.0 | 98.4 | 99.8 |
| 9 | 100.8 | 100.0 | 99.8 | 99.8 |
| 12 | 100.4 | 99.7 | 99.5 | 99.7 |
| 18 | 100.6 | 99.8 | - | - |

### Beispiel 3

### Pulverröntgendiagramme

Zur Charakterisierung der DOPC Kristallmodifikationen werden von diesen Substanzen Pulverröntgendiagramme (XRD-Beugungsspektren) aufgnommen. Zu Vergleichszwecken werden auch von lyophilisierten und wachsartigen DOPC-Varianten Pulverröntgendiagramme (XRD-Beugungsspektren) aufgenommen.

Für DOPC Kristallmodifikationen des Typ I ergeben sich für Lipide relativ gut aufgelöste Spektren mit mittelscharfen Banden. Die Spektren weisen auf hohe kristalline Anteile hin. Unter dem Polarisationsmikroskop sind keine amorphen Anteile sichtbar.

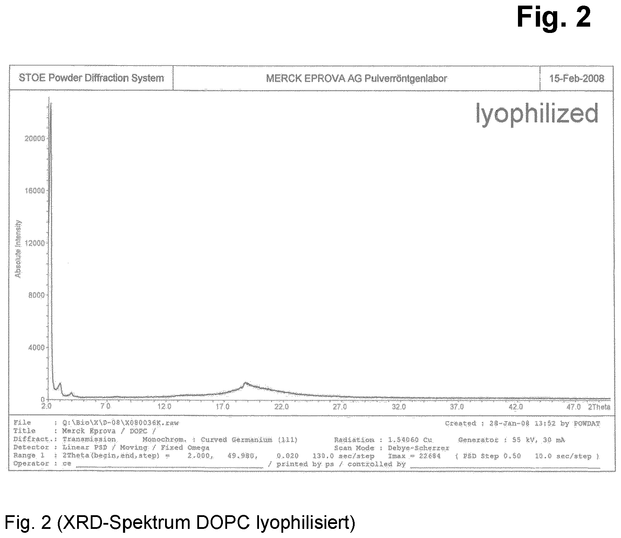

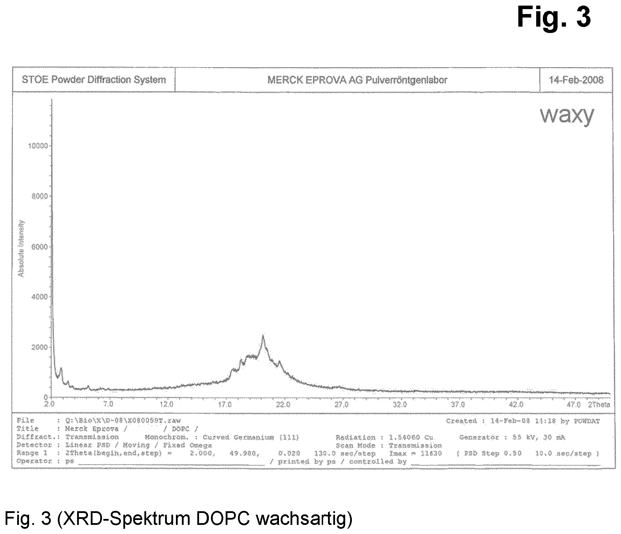

Ein Beispielspektrum ist in Abb. 1 (Typ I) ersichtlich. Zum Vergleich werden von kommerziell erhältlichen, amorphen Proben unter analogen Bedingungen ebenfalls Spektren aufgenommen und als Abb. 2 (lyophilisiert) und Abb. 3 (wachsartig) aufgeführt.

In Tabelle 2 sind ausgewählte 2-Theta-Werte für die DOPC Kristallmodifikation Typ I aufgelistet:

**Tabelle 2:**

| Typ | | ausgewählte 2-Theta-Werte |
|---|---|---|
| Typ I | (R)-DOPC | 3.6, 5.3, 7.1, 8.8, 11.0, 12.3, 15.3, 17.6, 18.3, 19.3, 20.4, 21.1, 21.7, 22.8 und 26.4 |

### Beispiel 4

### Modifikationen

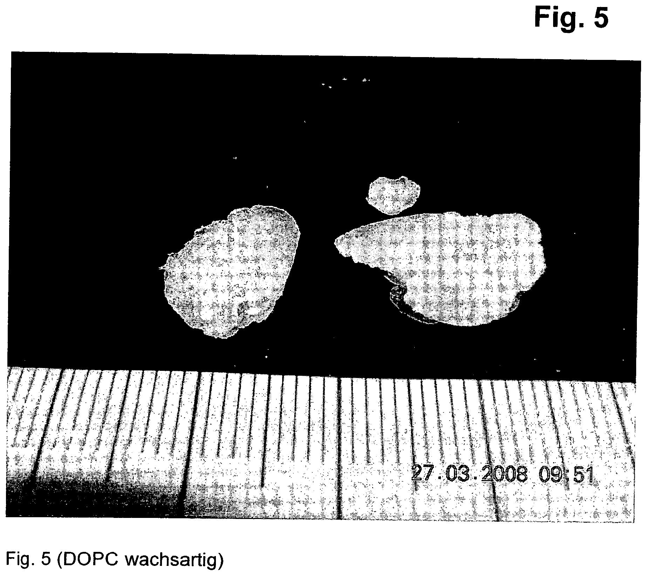

DOPC Kristallmodifikationen des Typ I fallen als körniges Schüttgut an (Abb. 4) während käufliche DOPC-Proben entweder als Lyophilisat oder als wachsartige Klumpen (Abb. 5) erhalten werden.

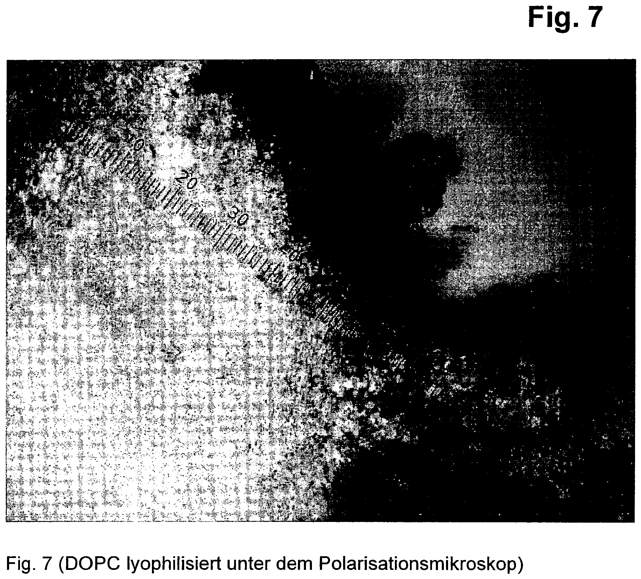

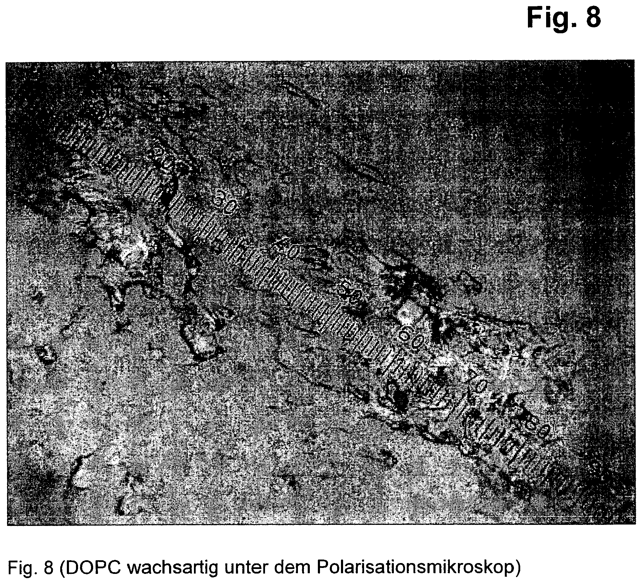

Unter dem Polarisationsmikroskop ist die DOPC Kristallmodifikation des Typ I eindeutig als kristallines Material erkennbar (Abb. 6), während die lyophilisierten (Abb. 7) bzw. wachsartigen Vergleichsmaterialien (Abb. 8) amorph erscheinen.

### Beispiel 5

### Schmelzverhalten

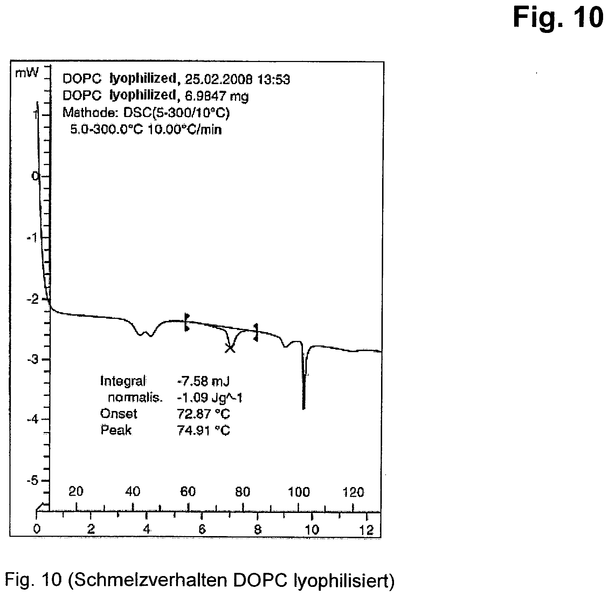

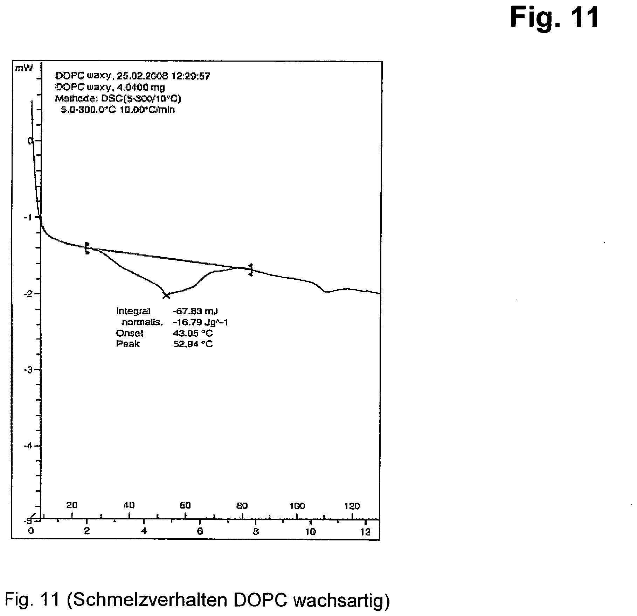

Differential Scanning Calorimetry (DSC) Messungen ergeben ebenfall deutliche Unterschiede für die DOPC Kristallmodifikation des Typ I (Abb. 9), und das lyophilisierte (Abb. 10) bzw. wachsartige Vergleichsmaterial (Abb. 11).

## Patentansprüche

1. Kristallines (R)- oder (S)-DOPC, **dadurch gekennzeichnet, dass** es 2-Theta-Werte (CuKa Strahlung) von 3.6, 5.3, 18.3, 19.3, und 21.7 ausweist.

2. Mischungen aus kristallinem (R)- und kristallinem (S)-DOPC gemäss Anspruch 1.

3. Kristallines (R)-DOPC gemäss Anspruch 1.

4. Kristallines (R)- oder (S)-DOPC gemäss Anspruch 1 mit 2-Theta-Werten (CuKa Strahlung) von 3.6, 5.3, 7.1, 8.8, 11.0, 12.3, 15.3, 17.6, 18.3, 19.3, 20.4, 21.1, 21.7, 22.8 und 26.4.

5. Kristallines (R)- oder (S)-DOPC gemäss Anspruch 1 mit 2-Theta-Werten (CuKa Strahlung) von 3.6, 5.3, 7.1, 12.3, 17.6, 18.3, 19.3, 21.1, 21.7 und.22.8.

6. Kristallines (R)- oder (S)-DOPC gemäss einem oder mehreren der Ansprüche 1 bis 5 mit einem Schmelzpunkt grösser als 60°C und einer Schmelzenthalpie grösser als 48 J/g.

7. Verfahren zur Herstellung von kristallinem (R)- oder (S)-DOPC gemäss mindestens einem der Ansprüche 1 bis 6, durch Kristallisation von (R)- oder (S)-DOPC aus einem oder mehreren aprotischen Lösungsmitteln.

8. Verfahren zur Herstellung von kristallinem (R)- oder (S)-DOPC gemäss Anspruch 7, **dadurch gekennzeichnet, dass** als aprotisches Lösungsmittel ein Ether, bevorzugt Tetrahydrofuran, Methyltetrahydrofuran oder Dioxan eingesetzt wird.

9. Verfahren zur Herstellung von kristallinem (R)- oder (S)-DOPC gemäss Anspruch 7, **dadurch gekennzeichnet, dass** als aprotisches Lösungsmittel ein Keton, bevorzugt Aceton, 2-Butanon, Methylisobutylketon, Methylisopropylketon eingesetzt wird.

10. Verfahren zur Herstellung von kristallinem (R)- oder (S)-DOPC gemäss Anspruch 7, **dadurch gekennzeichnet, dass** als aprotisches Lösungsmittel ein Nitril, bevorzugt Acetonitril eingesetzt wird.

11. Verfahren zur Herstellung von kristallinem (R)- oder (S)-DOPC gemäss Anspruch 7, **dadurch gekennzeichnet, dass** als polares Lösungsmittel ein Ester, ausgewählt aus der Gruppe Ethylformiat, Methylacetat, Ethylacetat, Propylacetat, Isopropylacetat, Butylacetat, Isobutylacetat, Dimethylcarbonat, Diethylcarbonat und 1,3-Dioxolidin-2-on oder deren Gemische eingesetzt wird.

12. Verfahren gemäß einem oder mehreren der Ansprüche 7 bis 11, **dadurch gekennzeichnet dass** ein aprotisches Medium im Gemisch mit einem Alkohol, ausgewählt aus der Gruppe Methanol, Ethanol, n-Propanol, iso-Propanol, n-Butanol, iso-Butanol, 2-Butanol, tert-Butanol, 3-Methyl-1-butanol, Ethylenglycol, Methoxyethanol und Ethoxyethanol oder deren Gemischen, verwendet wird.

13. Verfahren nach Anspruch 7, **dadurch gekennzeichnet, dass** die Kristallisation ohne vorherige Aufreinigung direkt aus der Reaktionslösung erfolgt.

14. Verfahren nach Anspruch 7, **dadurch gekennzeichnet, dass** eine Umkristallisation von amorphem oder kristallinem (R)- oder (S)-DOPC erfolgt.

15. Verwendung von Kristallmodifikationen von (R)- oder (S)-DOPC gemäss mindestens einem der Ansprüche 1 bis 6 allein oder gegebenenfalls im Gemisch mit anderen Lipiden als Bestandteil zur Herstellung von Arzneimitteln.

16. Pharmazeutische Zusammensetzung enthaltend kristallines (R)- oder (S)-DOPC gemäss mindestens einem der Ansprüche 1 bis 6 allein oder im Gemisch oder gegebenenfalls zusammen mit pharmazeutischen Wirkstoffen, Hilfsstoffen oder einem Lösemittel.

17. Pharmazeutische Zusammensetzung gemäss Anspruch 16, **dadurch gekennzeichnet, dass** als pharmazeutischer Wirkstoff ein Wirkstoff ausgewählt aus der Gruppe der Peptide, Nukleotide, Vakzine oder Zytostatika eingesetzt wird.

18. Pharmazeutische Zusammensetzung gemäss Anspruch 16, **dadurch gekennzeichnet, dass** sie Liposomen, Lipoplexe, Nanopartikel oder Mikroemulsionen aufweist.

## Claims

1. Crystalline (R)- or (S)-DOPC, **characterised in that** it has 2-theta values (CuKa radiation) of 3.6, 5.3, 18.3, 19.3 and 21.7.

2. Mixtures of crystalline (R)- and crystalline (S)-DOPC according to Claim 1.

3. Crystalline (R)-DOPC according to Claim 1.

4. Crystalline (R)- or (S)-DOPC according to Claim 1 having 2-theta values (CuKa radiation) of 3.6, 5.3, 7.1, 8.8, 11.0, 12.3, 15.3, 17.6, 18.3, 19.3, 20.4, 21.1, 21.7, 22.8 and 26.4.

5. Crystalline (R)- or (S)-DOPC according to Claim 1 having 2-theta values (CuKa radiation) of 3.6, 5.3, 7.1, 12.3, 17.6, 18.3, 19.3, 21.1, 21.7 and 22.8.

6. Crystalline (R)- or (S)-DOPC according to one or more of Claims 1 to 5 having a melting point of greater than 60°C and an enthalpy of melting of greater than 48 J/g.

7. Process for the preparation of crystalline (R)- or (S)-DOPC according to at least one of Claims 1 to 6, by crystallisation of (R)- or (S)-DOPC from one or more aprotic solvents.

8. Process for the preparation of crystalline (R)- or (S)-DOPC according to Claim 7, **characterised in that** the aprotic solvent employed is an ether, preferably tetrahydrofuran, methyltetrahydrofuran or dioxane.

9. Process for the preparation of crystalline (R)- or (S)-DOPC according to Claim 7, **characterised in that** the aprotic solvent employed is a ketone, preferably acetone, 2-butanone, methyl isobutyl ketone or methyl isopropyl ketone.

10. Process for the preparation of crystalline (R)- or (S)-DOPC according to Claim 7, **characterised in that** the aprotic solvent employed is a nitrile, preferably acetonitrile.

11. Process for the preparation of crystalline (R)- or (S)-DOPC according to Claim 7, **characterised in that** the polar solvent employed is an ester selected from the group ethyl formate, methyl acetate, ethyl acetate, propyl acetate, isopropyl acetate, butyl acetate, isobutyl acetate, dimethyl carbonate, diethyl carbonate and 1,3-dioxolidin-2-one or mixtures thereof.

12. Process according to one or more of Claims 7 to 11, **characterised in that** use is made of an aprotic medium in a mixture with an alcohol selected from the group methanol, ethanol, n-propanol, isopropanol, n-butanol, isobutanol, 2-butanol, tert-butanol, 3-methyl-1-butanol, ethylene glycol, methoxyethanol and ethoxyethanol or mixtures thereof.

13. Process according to Claim 7, **characterised in that** the crystallisation is carried out directly from the reaction solution without prior purification.

14. Process according to Claim 7, **characterised in that** a recrystallisation of amorphous or crystalline (R)- or (S)-DOPC is carried out.

15. Use of crystal modifications of (R)- or (S)-DOPC according to at least one of Claims 1 to 6, alone or optionally in a mixture with other lipids, as constituent for the preparation of medicaments.

16. Pharmaceutical composition comprising crystalline (R)- or (S)-DOPC according to at least one of Claims 1 to 6, alone or in a mixture or optionally together with pharmaceutical active compounds, assistants or a solvent.

17. Pharmaceutical composition according to Claim 16, **characterised in that** the pharmaceutical active compound employed is an active compound selected from the group of the peptides, nucleotides, vaccines and cytostatics.

18. Pharmaceutical composition according to Claim 16, **characterised in that** it comprises liposomes, lipoplexes, nanoparticles or microemulsions.

## Revendications

1. (R)- ou (S)-DOPC cristalline, **caractérisée en ce qu'**elle présente des valeurs 2-thêta (rayonnement CuKa) de 3.6, 5.3, 18.3, 19.3 et 21.7.

2. Mélanges de (R)-DOPC cristalline et de (S)-DOPC cristalline selon la revendication 1.

3. (R)-DOPC cristalline selon la revendication 1.

4. (R)- ou (S)-DOPC cristalline selon la revendication 1, présentant des valeurs 2-thêta (rayonnement CuKa) de 3.6, 5.3, 7.1, 8.8, 11.0, 12.3, 15.3, 17.6, 18.3, 19.3, 20.4, 21.1, 21.7, 22.8 et 26.4.

5. (R)- ou (S)-DOPC cristalline selon la revendication 1, présentant des valeurs 2-thêta (rayonnement CuKa) de 3.6, 5.3, 7.1, 12.3, 17.6, 18.3, 19.3, 21.1, 21.7 et 22.8.

6. (R)- ou (S)-DOPC cristalline selon l'une ou plusieurs parmi les revendications 1 à 5, présentant un point de fusion supérieur à 60°C et une enthalpie de fusion supérieure à 48 J/g.

7. Procédé de préparation de (R)- ou (S)-DOPC cristalline selon au moins l'une des revendications 1 à 6, par cristallisation de (R)- ou (S)-DOPC dans un ou plusieurs solvants aprotiques.

8. Procédé de préparation de (R)- ou (S)-DOPC cristalline selon la revendication 7, **caractérisé en ce que** le solvant aprotique employé est un éther, préférablement le tétrahydrofurane, le méthyltétrahydrofurane ou le dioxane.

9. Procédé de préparation de (R)- ou (S)-DOPC cristalline selon la revendication 7, **caractérisé en ce que** le solvant aprotique employé est une cétone, préférablement l'acétone, la 2-butanone, la méthyliso-butylcétone ou la méthylisopropylcétone.

10. Procédé de préparation de (R)- ou (S)-DOPC cristalline selon la revendication 7, **caractérisé en ce que** le solvant aprotique employé est un nitrile, préférablement l'acétonitrile.

11. Procédé de préparation de (R)- ou (S)-DOPC cristalline selon la revendication 7, **caractérisé en ce que** le solvant polaire employé est un ester choisi dans le groupe constitué par le formiate d'éthyle, l'acétate de méthyle, l'acétate d'éthyle, l'acétate de propyle, l'acétate d'isopropyle, l'acétate de butyle, l'acétate d'isobutyle, le carbonate de diméthyle, le carbonate de diéthyle et la 1,3-dioxolidin-2-one ou des mélanges de ceux-ci.

12. Procédé selon l'une ou plusieurs parmi les revendications 7 à 11, **caractérisé en ce qu'**on utilise un milieu aprotique en mélange avec un alcool choisi dans le groupe constitué par le méthanol, l'éthanol, le n-propanol, l'isopropanol, le n-butanol, l'isobutanol, le 2-butanol, le tertio-butanol, le 3-méthyl-1-butanol, l'éthylène glycol, le méthoxyéthanol et l'éthoxyéthanol ou des mélanges de ceux-ci.

13. Procédé selon la revendication 7, **caractérisé en ce que** la cristallisation est effectuée directement dans la solution réactionnelle sans purification préalable.

14. Procédé selon la revendication 7, **caractérisé en ce qu'**une recristallisation de (R)- ou (S)-DOPC amorphe ou cristalline est effectuée.

15. Utilisation de modifications cristallines de (R)- ou (S)-DOPC selon au moins l'une des revendications 1 à 6, seules ou éventuellement en mélange avec d'autres lipides, comme constituant pour la préparation de médicaments.

16. Composition pharmaceutique comprenant de la (R)- ou (S)-DOPC cristalline selon au moins l'une des revendications 1 à 6, seule ou en mélange ou éventuellement conjointement avec des composés actifs pharmaceutiques, des auxiliaires ou un solvant.

17. Composition pharmaceutique selon la revendication 16, **caractérisée en ce que** le composé actif pharmaceutique employé est un composé actif choisi dans le groupe constitué par les peptides, les nucléotides, les vaccins et les cytostatiques.

18. Composition pharmaceutique selon la revendication 16, **caractérisée en ce qu'**elle comprend des liposomes, des lipoplexes, des nanoparti-cules ou des microémulsions.
